# EUROPEAN PATENT APPLICATION

(11) **EP 4 268 785 A1**
(43) Date of publication of application: **01.11.2023**
(21) Application number: 21910172.2
(22) Date of filing: 30.11.2021
(51) Int. Cl.: A61H 3/00

(54) **WALKING ASSISTANCE DEVICE AND WALKING ASSISTANCE METHOD**

(30) Priority: 25.12.2020 JP 2020216043
(71) Applicant: CYBERDYNE INC., Tsukuba-shi, Ibaraki 305-0818 (JP)
(72) Inventor: SANKAI, Yoshiyuki, Tsukuba-shi, Ibaraki 305-0818 (JP)
(74) Representative: Mewburn Ellis LLP
(86) International application number: PCT/JP2021/043987
(87) International publication number: WO 2022/138020

(57) **Abstract**

A wearer can smoothly perform the plantar-dorsiflexion motion of the toes in coordination with physical stimulating power according to one's own intention while maintaining the left/right balance of one's gait by adjusting the hybrid ratio of voluntary control and autonomous control so that the left/right balance of the wearer's gait will fall within a predetermined range.

## Description

### TECHNICAL FIELD

The present invention relates to a walking support device and a walking support method and, for example, can be suitably applied to patients suffering from drop foot symptoms.

### BACKGROUND ART

Patients who have suffered a cerebral vascular disease such as apoplexy, cerebral hemorrhage or cerebral infarction, or a spinal cord injury, are often left with flaccid or spastic paralysis in the lower limb. Flaccid paralysis causes a symptom known as a "drop foot" in which the toes of the lower limb become plantarflexed more than those of able-bodied persons.

For a patient suffering from the symptom of a drop foot, the dorsal flexion of the ankle is difficult due to the flaccidity of the dorsiflexor muscles or the accentuation of the plantar flexor muscles of the ankle. When this patient engages in a walking motion, since the toes become plantarflexed in the pre-swing of swinging the paralyzed lower limb forward and the motion continues to the terminal swing in that state, the patient may fall since the toes will come into contact with the floor before the heel.

A patient suffering from the symptom of a drop foot tends to drag one's feet during the swing phase of the walking motion, and, because the patient normally attempts to compensate the dragging of one's feet by raising the corresponding hip or with the swinging of the corresponding leg in a circular motion, there is a risk of the patient falling on a frequent basis as a result of not being able to smoothly shift one's weight.

Conventionally, a proposal has been made for having a patient suffering from the symptom of a drop foot wear a functional electrical stimulation (FES) harness for gait adjustment and thereby facilitating the walking motion of the patient by selectively applying electrical stimulation to a part of the neuromuscular system of the leg (see PTL 1).

Also proposed is a functional electrical stimulation system which detects the gait phase and evaluates the walking quality from the path of the feet of a patient suffering from the symptom of a drop foot, and correcting the stimulation pattern based thereon (see PTL 2).

### CITATION LIST

### PATENT LITERATURE

[PTL 1] Japanese Unexamined Patent Application Publication (Translation of PCT Application) No. 2019-505290
[PTL 2] Japanese Unexamined Patent Application Publication (Translation of PCT Application) No. 2017-517370

### SUMMARY OF THE INVENTION

### PROBLEMS TO BE SOLVED BY THE INVENTION

Meanwhile, with the functional electrical stimulation harness shown in PTL 1, stimulation is applied to cause the dorsiflex or plantarflex of the patient's foot by guiding or operating a multichannel electrical stimulation current to a part of the neuromuscular system of the patient's lower limb by associating it with the patient's walking event (heel off event), walking speed and inclination or floor reaction force of the feet.

Moreover, with the functional electrical stimulation system shown in PTL 2, the electrical stimulation pattern is corrected when the walking quality evaluated from the path of the patient's feet is less than a certain threshold.

Nevertheless, in PTL 1 and PTL 2, the patient's walking state is merely detected using a sensor group consisting of motion sensors and a gyroscope, and, since the patient's walking motion is adjusted to be optimal based only on the detection result of such sensor group, there is a possibility that it would be practically inadequate for a patient, who is suffering from the symptom of a drop foot, to smoothly swing his/her legs in real-time according to one's intention when engaging in a walking motion.

The present invention was devised in consideration of the foregoing points, and an object of this invention is to propose a walking support device and a walking support method which enable the wearer to smoothly perform the plantar-dorsiflexion motion of the toes according to one's own intention.

### MEANS TO SOLVE THE PROBLEMS

In order to achieve the foregoing object, the present invention provides a walking support device, comprising: a load measurement part which is attached to each inner sole part of a pair of left and right shoes of a wearer, and which measures a load applied on an underside of the wearer's feet; a center of gravity calculation part which calculates a center of gravity of each of the feet based on a change of each load measured with the load measurement part; a foot motion detection part which is mounted on a predetermined position of each of the shoes, and detects an acceleration and/or an angular rate during movement of the foot; a movement locus calculation part which calculates a movement locus of each of the feet based on each acceleration and/or each angular rate detected by the foot motion detection part; a gait recognition part which recognizes a gait of the wearer based on the calculated center of gravity and movement locus of each of the feet; a biosignal detection part which has an electrode group for detecting a biosignal of the wearer each disposed on the wearer's body surface area with reference to a joint associated with the wearer's walking motion; a stimulation application part which has a terminal group for applying physical stimulation to a body surface of a paralysis site each disposed at the paralysis site centered around dorsiflexor muscles and plantar flexor muscles of the wearer's ankle; a voluntary control part which voluntarily controls the stimulation application part so as to generate stimulating power according to the wearer's intention based on the biosignal detected by the biosignal detection part; an autonomous control part which autonomously controls the stimulation application part so as to cause the ankle to dorsiflex or plantarflex based on contraction of the dorsiflexor muscles or the plantar flexor muscles of the wearer based on the wearer's walking cycle obtained from a recognition result of the gait recognition part; and an optimal stimulation adjustment part which adjusts the voluntary control by the voluntary control part and the autonomous control by the autonomous control part so that a left/right balance of the wearer's gait recognized by the gait recognition part will fall within a predetermined range.

Consequently, with the walking support device, without the wearer having to wear a drive mechanism such as an actuator, it is possible to voluntarily control the stimulation application part so as to generate stimulating power according to the wearer's intention and autonomously control the stimulation application part so as to cause the ankle to dorsiflex or plantarflex based on contraction of the dorsiflexor muscles or the plantar flexor muscles of the wearer based on the wearer's walking cycle. Furthermore, by adjusting the hybrid ratio of the voluntary control and the autonomous control so that a left/right balance of the wearer's gait will fall within a predetermined range, the wearer can smoothly perform the plantar-dorsiflexion motion of the toes in coordination with the physical stimulating power according to one's own intention while maintaining the left/right balance of one's gait.

Moreover, in the present invention, the stimulation application part adjusts at least one among strength level, pattern and time of applying stimulation to the wearer's paralysis site according to control of the voluntary control part and/or the autonomous control part. Consequently, with the walking support device, adjustment can be made so that the plantar-dorsiflexion motion of the wearer's toes is performed smoothly while maintaining the left/right balance of the wearer's gait at the same time.

In addition, the present invention further comprises a sound collecting microphone which is provided to the shoe on a side where the wearer's ankle has a paralysis site, and collects sound of footsteps of the shoe, and the optimal stimulation adjustment part corrects the hybrid ratio based on the sound of footsteps collected with the sound collecting microphone. Consequently, with the walking support device, by comprehending the status of a floor surface while collecting the sound when the wearer's toes contact the floor surface, adjustment can be made even more accurately so that the plantar-dorsiflexion motion of the wearer's toes is performed smoothly.

In addition, the present invention further comprises a vibration detection part which is provided to the shoe on a side where the wearer's ankle has a paralysis site, and detects vibration from a floor surface while the wearer is walking, and the optimal stimulation adjustment part corrects the hybrid ratio based on the vibration detected with the vibration detection part. Consequently, with the walking support device, by comprehending the status of a floor surface while detecting the vibration from the floor surface that reaches the wearer's shoes, adjustment can be made even more accurately so that the plantar-dorsiflexion motion of the wearer's toes is performed smoothly.

The present invention additionally provides a walking support method, comprising: a first process of calculating a center of gravity of each of a wearer's feet based on a change of a load applied to an underside of each of the feet; a second process of calculating a movement locus of each of the feet based on an acceleration and/or an angular rate during movement of each of the feet; a third process of recognizing the wearer's gait based on the calculated center of gravity and movement locus of each of the feet; a fourth process of detecting the wearer's biosignal using an electrode group each disposed on the wearer's body surface area with reference to a joint associated with the wearer's walking motion; a fifth process of applying physical stimulation to a body surface of a paralysis site each disposed at the paralysis site centered around dorsiflexor muscles and plantar flexor muscles of the wearer's ankle; a sixth process of voluntarily controlling the application of stimulation in the fifth process so as to generate stimulating power according to the wearer's intention based on the biosignal detected in the fourth process; a seventh process of autonomously controlling the application of stimulation in the fifth process so as to cause the ankle to dorsiflex or plantarflex based on contraction of the dorsiflexor muscles or the plantar flexor muscles of the wearer based on the wearer's walking cycle obtained from a recognition result of the third process; and an eighth process of adjusting the voluntary control based on the sixth process and the autonomous control based on the seventh process so that a left/right balance of the wearer's gait recognized in the third process will fall within a predetermined range.

Consequently, with the walking support method, without the wearer having to wear a drive mechanism such as an actuator, it is possible to voluntarily control the application of stimulation so as to generate stimulating power according to the wearer's intention and autonomously control the application of stimulation so as to cause the ankle to dorsiflex or plantarflex based on contraction of the dorsiflexor muscles or the plantar flexor muscles of the wearer based on the wearer's walking cycle. Furthermore, by adjusting the hybrid ratio of the voluntary control and the autonomous control so that a left/right balance of the wearer's gait will fall within a predetermined range, the wearer can smoothly perform the plantar-dorsiflexion motion of the toes in coordination with the physical stimulating power according to one's own intention while maintaining the left/right balance of one's gait.

### ADVANTAGEOUS EFFECTS OF THE INVENTION

According to the present invention, it is possible to realize a walking support device and a walking support method which enable the wearer to smoothly engage in the plantar-dorsiflexion motion of the toes according to one's own intention while maintaining the left/right balance of the gait.

### BRIEF DESCRIPTION OF DRAWINGS

[Fig. 1]

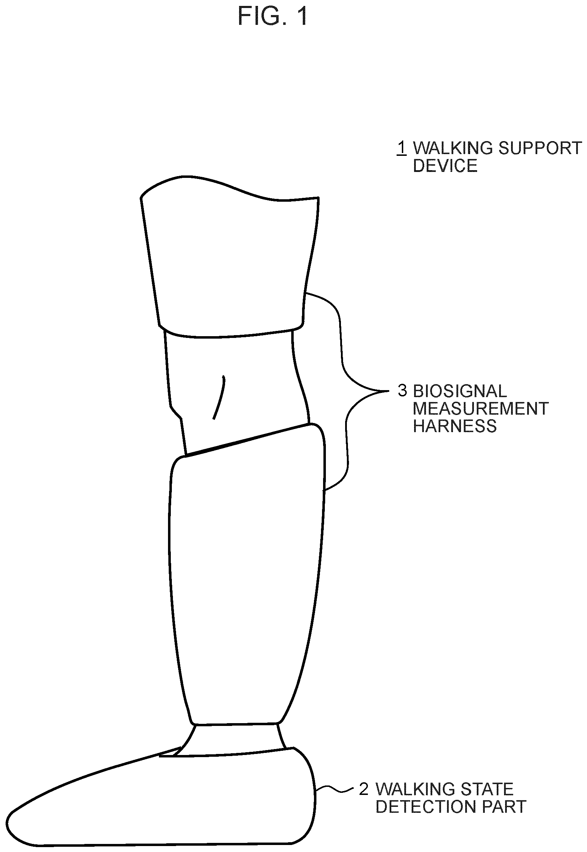

   Fig. 1 is a schematic diagram showing the overall configuration of the walking support device according to this embodiment.
[Fig. 2]

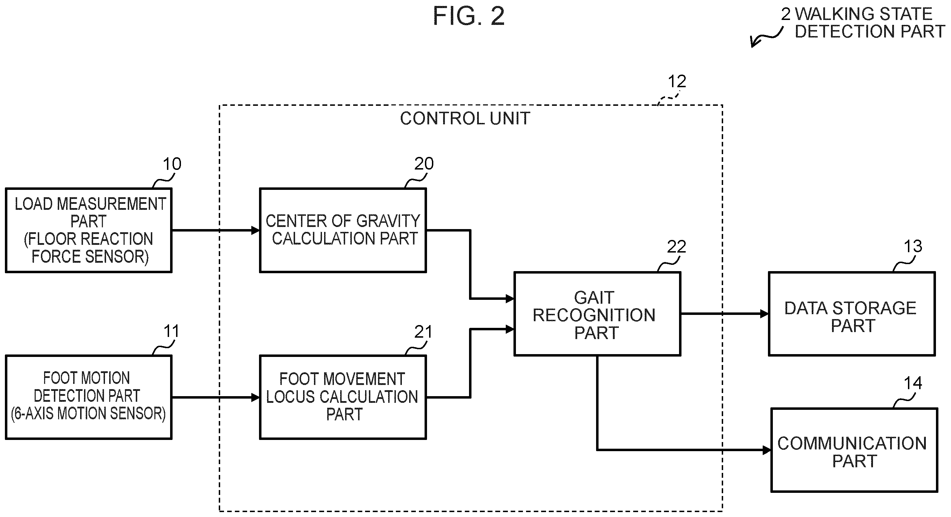

   Fig. 2 is a block diagram showing the configuration of the walking state detection part depicted in Fig. 1.
[Fig. 3]

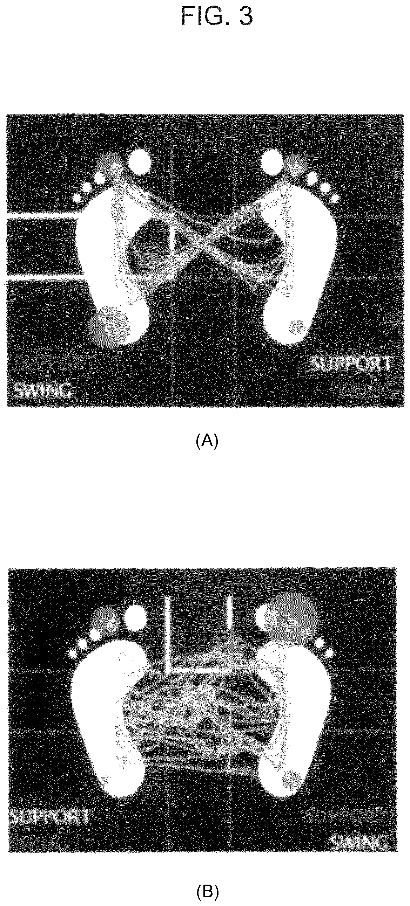

   Fig. 3 is a diagram showing the Lissajous figure reflecting the locus of the center of floor reaction force.
[Fig. 4]

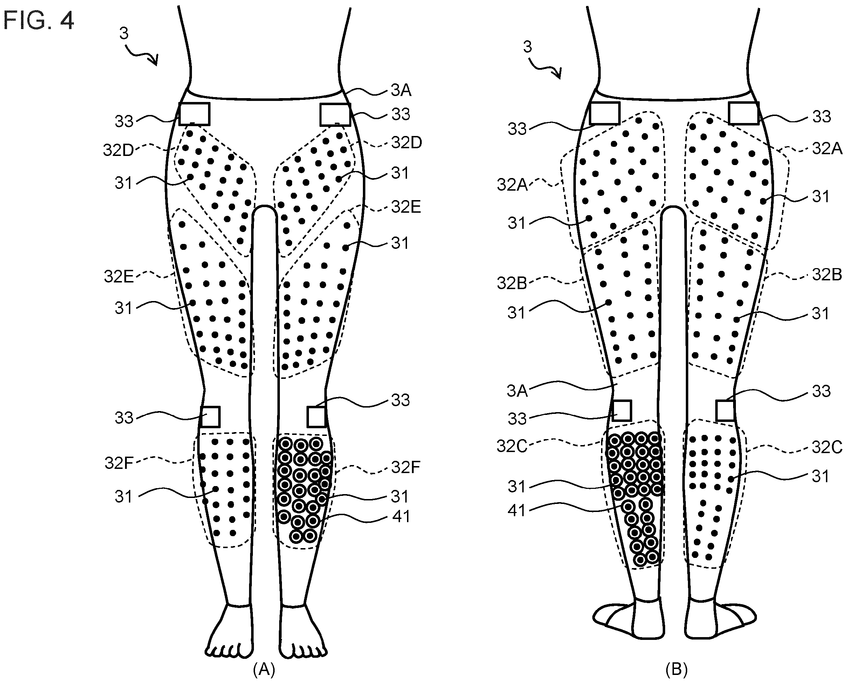

   Fig. 4 is a diagram showing an example of the biosignal measurement harness for the lower body.
[Fig. 5]

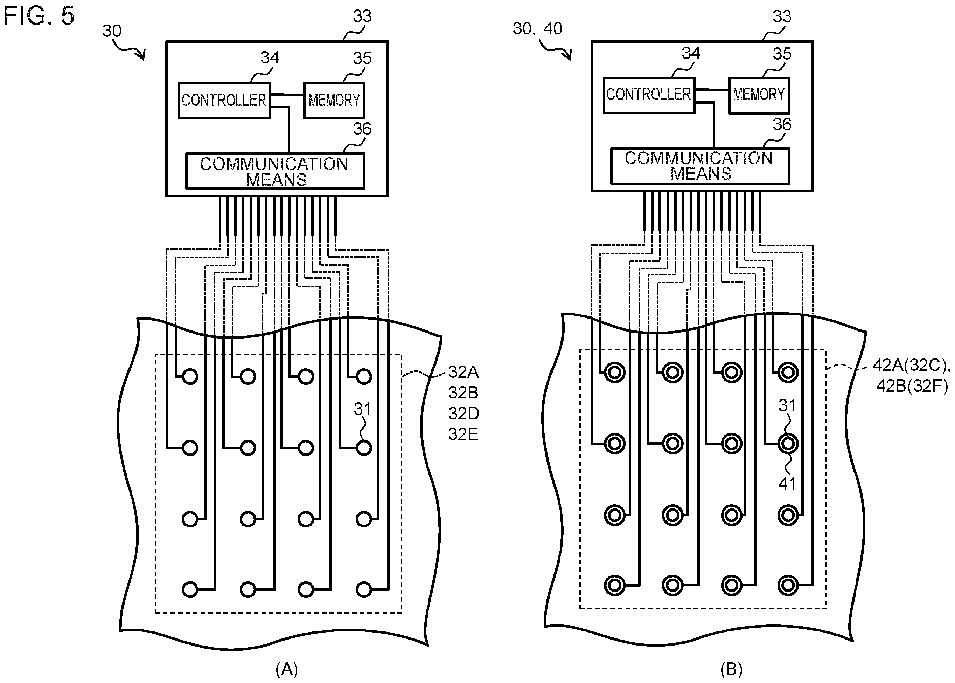

   Fig. 5 is a diagram showing an example of the measurement module in the biosignal measurement harness depicted in Fig. 4.
[Fig. 6]

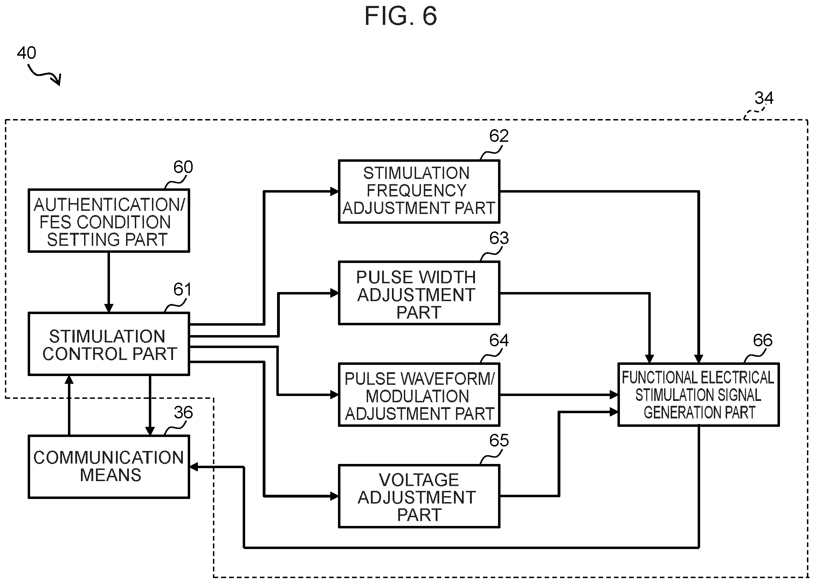

   Fig. 6 is a block diagram showing the configuration of the controller in the measurement module having the stimulation application part.
[Fig. 7]

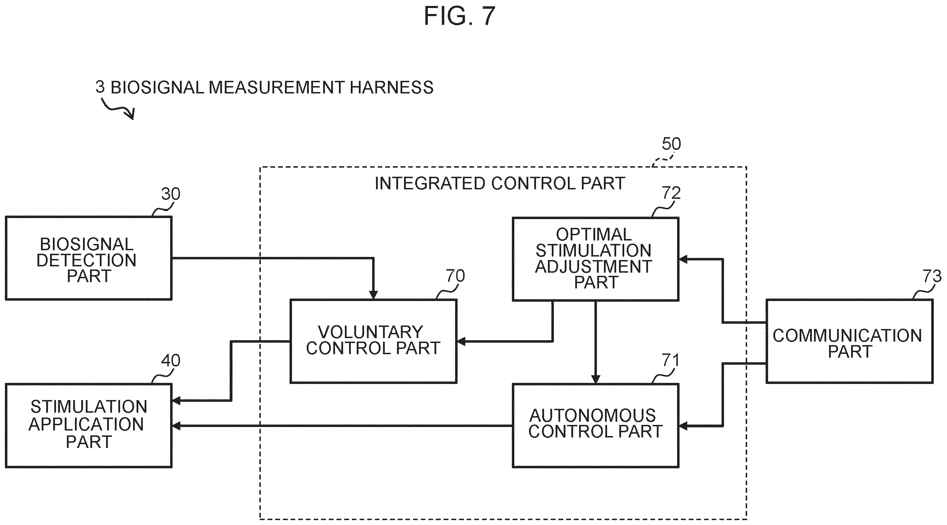

   Fig. 7 is a block diagram showing the configuration of the control system in the biosignal measurement harness.

### DESCRIPTION OF EMBODIMENTS

An embodiment of the present invention is now explained in detail with reference to the appended drawings.

### (1) Configuration of the walking support device according to this embodiment

Fig. 1 shows a walking support device 1 according to this embodiment, and includes a walking state detection part 2 configured from a pair of shoes mounted with various sensor groups, and a biosignal measurement harness 3 to be worn on a wearer's left and right lower limbs.

As shown in Fig. 2, the walking state detection part 2 is attached to each inner sole part of a pair of left and right shoes of the wearer, and includes a load measurement part 10 which measures a load applied to an underside of the wearer's feet, and a foot motion detection part 11 which is mounted on a predetermined position of each of the shoes, and detects an acceleration and/or an angular rate during the movement of the foot.

Specifically, the load measurement part 10 is configured by the shoe sole being covered by floor reaction force sensors. Moreover, the foot motion detection part 11 is configured from a 6-axis motion sensor formed from a 3-axis acceleration sensor and a 3-axis angular rate sensor (gyro sensor) attached to the upper center part of each shoe.

The walking state detection part 2 includes a control unit 12 which processes information obtained from the load measurement part 10 and the foot motion detection part 11, a data storage part 13 which stores data processed by the control unit 12, and a communication part 14 which sends and receives signals to and from the biosignal measurement harness 3 described later.

The control unit 12 is provided with a center of gravity calculation part 20 which controls the center of gravity of each foot based on a change of each load measured with the load measurement part 10, a foot movement locus calculation part 21 which calculates the movement locus of each foot based on each acceleration and/or each angular rate detected by the foot motion detection part 11, and a gait recognition part 22 which recognizes the wearer's gait based on the calculated center of gravity and movement locus of each foot.

Specifically, the center of gravity calculation part 20 receives the measurement result of the detected load on the underside of each foot from the floor reaction force sensor, and calculates the center of gravity (position of center of floor reaction force) of each foot based on the change in each load. As the configuration of the center of gravity calculation part 20, it is desirable to incorporate the same configuration as the center of gravity detection device disclosed in Japanese Patent No. 4997614 in which the right holder is the same as the present applicant.

The foot movement locus calculation part 21 calculates the movement locus of each foot by receiving the detected acceleration and the angular rate during the movement of each foot from the 6-axis motion sensor consisting of the 3-axis acceleration sensor and the 3-axis angular rate sensor.

The gait recognition part 22 operates, as the locus of the center of gravity (center of floor reaction force), changes in the center of gravity of each foot based on the center of gravity of each foot calculated by the center of gravity calculation part 20, and the movement locus of each foot calculated by the foot movement locus calculation part 21.

The data storage part 13 stores data representing the locus of the center of gravity (center of floor reaction force) of each foot while the wearer is walking during normal times, and this can be expressed as a Lissajous figure (figure in which the vibration waveform in units of the walking cycle is expressed as a closed curve on the XY plane). The expression of "during normal times" represents a state upon assuming that the wearer's walking state is a certain walking cycle and the left/right balance (mainly the wearer's stride) is equal.

As shown in Fig. 3(A), with the Lissajous figure during normal times, the locus of the center of gravity of each foot draws a regular circle, and becomes substantially constant. This is because each foot, while the wearer is walking, is repeating the same movement, and the shifting of the center of gravity becomes constant.

Meanwhile, as shown in Fig. 3(B), if the wearer contracts a cerebral vascular disease or suffers a spinal cord injury and develops the symptom of a drop foot, changes in the center of gravity of each foot will occur, the locus of the center of gravity will become an irregular circle, and the left/right balance will collapse.

The gait recognition part 22 reads the data representing the locus of the center of gravity during the wearer's normal times stored in the data storage part 13, compares it with the wearer's current locus of the center of gravity of each foot, and evaluates the wearer's gait based on the comparison result.

The data storage part 13 stores the data representing the closed curve (Lissajous figure) of the two-dimensional coordinate system, in which the locus of the center of gravity (center of floor reaction force) of each foot is indicated as a vibration waveform in units of the walking cycle, regarding the wearer's walk during normal times.

The gait recognition part 22 calculates the waveform pattern representing the closed curve data of the foregoing two-dimensional coordinate system concerning the calculated locus of the center of gravity (center of floor reaction force) of each foot of the wearer, and thereafter reads and compares the waveform pattern representing the curve data during normal times.

Subsequently, the gait recognition part 22 determines the closest characteristic of the difference in the waveform patterns as the comparison result based on the waveform pattern (curve data) unique to the wearer stored in the data storage part 13, and determines the degree of difference in the waveform patterns (that is, level of uniqueness of changes in the center of gravity of each foot) as the recognition result of the wearer's gait (status of deviation from normal times).

The walking state detection part 2 wirelessly communicates the recognition result of the gait recognition part 22 to the biosignal measurement harness 3 via the communication part 14. The communication part 14 of the walking state detection part 2 and the communication part (not shown) of the biosignal measurement harness 3 is connected with a short-range wireless communication system such as Bluetooth (registered trademark) or RF-ID, and data is sent and received therebetween.

The biosignal measurement harness 3 in this embodiment has a structure of disposing only the biosignal detection part 30 (Fig. 7) which detects the wearer's biosignal on the healthy leg, and disposing, in addition to the biosignal detection part 30, the stimulation application part 40 (Fig. 7) for applying physical stimulation to the paralysis site to the leg on the side where the wearer's ankle has the paralysis site (leg on the side having the symptom of a drop foot).

The biosignal measurement harness 3 according to this embodiment has the same configuration described in Japanese Patent Registration No. 5409637 and Japanese Patent Registration No. 6145663 by the present inventors and use the same principle. The biosignal measurement harness is shown in Fig. 4(A) and Fig. 4(B).

The biosignal measurement harness 3 is formed to cover the wearer's body surface, and includes a harness body 3A to be borne by the wearer, and the inner surface of the harness body 3A (face that comes into contact with the wearer's body surface in a worn state) is provided with the biosignal detection part 30 having an electrode group for detecting a biopotential signal at at least one position that can measure the biosignals from the wearer's body.

In Fig. 4(A) and Fig. 4(B), the biosignal measurement harness 3 of this embodiment corresponds to a case where the right leg of the wearer's lower limb is healthy and the left leg shows the symptom of a drop foot. In order words, with the biosignal measurement harness 3 of this embodiment, the biosignal detection part 30 is disposed on the right leg, and the stimulation application part 40 is disposed on the left leg, in addition to the biosignal detection part 30, centered around the part below the knee. Fig. 4(A) shows the lower body of the wearer's front side, and Fig. 4(B) shows the lower body on the wearer's back side.

With the harness body 3A, a plurality of electrodes 31 in the biosignal detection part 30 is each disposed on the wearer's body surface area based on the joint associated with the wearer's walking motion. The respective electrodes 31 are arranged in even intervals along the flow of the muscles of the wearer's legs.

In order words, a plurality of electrodes 31 is arranged along the flow of the gluteus maximus muscles at the part (electrode group 32A) corresponding to the wearer's hips. Moreover, similarly, a plurality of electrodes 31 is arranged along the flow of the biceps femoris muscles, the semimembranosus muscles, and the semitendinosus muscles at the part (electrode group 32B) corresponding to the back side of the wearer's thigh, and arranged along the triceps surae muscles at the part (electrode group 32C) corresponding to the calf.

Moreover, a plurality of electrodes 31 is arranged along the flow of the adductor longus muscles and the iliopsoas muscles at the part (electrode group 32D) corresponding to the front side of the hipjoint, arranged along the flow of the quadriceps femoris muscles at the part (electrode group 32E) corresponding to the front side of the thigh, and arranged along the flow of the tibialis anterior muscles, the soleus muscles, and the extensor digitorum longus muscles at the part (electrode group 32F) corresponding to the shin.

Here, with the biosignal measurement harness 3, a stimulation application part 40 having a terminal group which comes into contact with the wearer's body surface and applies electrical stimulation according to the biostimulation signal is provided to the part below the knee of the wearer's left leg (part of the ankle with the paralysis site), and a plurality of pairs of the terminal 41 and the foregoing electrode 31 is disposed in a matrix.

Here, Fig. 5(A) is a diagram showing an example of the biosignal detection part 30 provided to the harness body 3A. The biosignal detection part 30 is configured from electrode groups 32A to 32F formed from a plurality of biosignal electrodes 31 and a measurement module 33 connected to the electrode groups 32A to 32F.

Note that, since all electrode groups 32A to 32F provided to the harness body 3A are similarly connected to the measurement module 33, the electrode groups 32A to 32F are taken as an example and explained below. The respective electrodes 31 are provided in a mutually insulated state, and they are each connected to the measurement module 33 via conductive wiring. An address is assigned to each of these electrodes 31.

A plurality of electrodes 31 configuring the electrode groups 32A to 32F is connected to the measurement module 33, and the measurement module 33 comprises a measurement module controller 34 which selects at least two electrodes 31 among these electrodes 31 and acquires a biosignal by obtaining the difference between the detection signals detected by these selected electrodes 31, a memory 35 which records the acquired biosignals, and a communication means 36 which transmits the sequentially acquired biosignals and/or the biosignals recorded in the memory 35 to the outside (integrated control part 50 shown in Fig. 7 described later). Note that the measurement module 33 is provided for each of the electrode groups 32A to 32F, and the electrodes 31 of each of the electrode groups 32A to 32F are connected thereto.

The measurement module controller 34 includes an electronic circuit which sequentially selects at least two electrodes 31 from a plurality of electrodes 31 that have been connected according to the command signal input via the communication means 36, and can acquire the biosignals between these two selected electrodes.

Moreover, the measurement module controller 34 further includes a filter for removing or extracting predetermined frequency components from the thus acquired biosignals, and signal processing means such as an amplifier which amplifies the acquired biosignals. The thus acquired biosignals are output from the measurement module controller 34 to the communication means 36 and/or the memory 35.

When the measurement module controller 34 is to select the electrode 31, it may sequentially perform operations so that each electrode 31 is selected in a predetermined order, or select the electrode 31 corresponding to the address designated by a designation signal based on the designation signal input via the communication means 36.

The communication means 36 is configured from a thin antenna, and a communication circuit connected to the antenna. The communication means 36 sends to the integrated control part 50 (Fig. 7), via the antenna, measurement information including a signal including the biosignal output from the measurement module controller 34 and information such as the address indicating the position information of the electrode 31 that detected the biosignal and/or the biosignal read from the memory 35 and information such as the address indicating the position information of the electrode 31 that detected the biosignal.

The integrated control part 50 generates a designation signal for designating which electrode 31 to select from the electrode groups 32A to 32F provided to the harness body 3A, and a signal for the start or end of the acquisition of data, and sends these signals to the measurement module controller 34 via an antenna. The measurement module controller 34 selects and operates the designated electrode 31 according to the received signals.

According to the biosignal measurement harness 3 described above, by detecting the biosignals with a plurality of electrodes 31, biosignals can be measured at each of a plurality of points in the area where the electrode groups 32A to 32F are disposed. By mapping the measured data of each point according to the address assigned to each electrode 31, the distribution of the biosignals of the wearer's body can be measured. The stimulation application part 40 can thereby be voluntarily controlled by accurately reflecting the wearer's intention based on the measurement result.

Furthermore, in the biosignal measurement harness 3, the stimulation application part 40 is disposed at each paralysis site centered around the dorsiflexor muscles (mainly the tibialis anterior muscles, extensor digitorum longus muscles and so on) and the plantar flexor muscles (mainly the gastrocnemius muscles, soleus muscles, plantar flexor muscles and so on) of the wearer's ankle, and has a terminal group for applying electrical stimulation to the body surface of the paralysis site.

The stimulation application part 40 is configured from a device that causes the muscles of the paralysis site to contract based on functional electrical stimulation (FES). With this stimulation application part 40, the body surface area corresponding to the dorsiflexor muscles and the plantar flexor muscles centered around the ankle is optimal as the body surface area to which electrical stimulation is to be applied to the wearer.

As shown in Fig. 5(B), to which the same reference numeral is affixed for parts corresponding to Fig. 5(A), the stimulation application part 40 is configured from a terminal group 42A formed from a plurality of electrical stimulation terminals 41, and a measurement module 33 of the foregoing biosignal detection part 30 connected to the terminal group 42A. The terminal group 42A of the stimulation application part 40 corresponds to the electrode group 32C of the biosignal detection part 30, and the terminal group 42B of the stimulation application part 40 corresponds to the electrode group 32F of the biosignal detection part 30. Note that, in this embodiment, while the measurement module 33 concurrently serves as the biosignal detection part 30 and the stimulation application part 40, these may also be provided separately.

As shown in Fig. 6, the measurement module controller 34 in the measurement module 33 includes an authentication/FES condition setting part 60, a stimulation control part 61, a stimulation frequency adjustment part 62, a pulse width adjustment part 63, a pulse waveform/modulation adjustment part 64, a voltage adjustment part 65, and a functional electrical stimulation signal generation part 66.

The authentication/FES condition setting part 60 sets the authentication data (ID, password) of the doctor or physical therapist that was input based on operations of the input operation part (not shown), and the FES conditions (stimulation frequency, pulse width, pulse waveform/modulation, voltage and other conditions) input based on the diagnosis of the doctor or physical therapist.

The stimulation control part 61 sends the signals of the functional electrical stimulation received from the integrated control part 50 (Fig. 7) via the communication means 36, together with the setting results of the authentication/FES condition setting part 60, to the stimulation frequency adjustment part 62, the pulse width adjustment part 63, the pulse waveform/modulation adjustment part 64, and the voltage adjustment part 65.

The stimulation frequency adjustment part 62 sets the stimulation frequency of the functional electrical stimulation to the frequency set by the authentication/FES condition setting part 60. The pulse width adjustment part 63 adjusts the pulse width of the functional electrical stimulation to the pulse width set by the authentication/FES condition setting part 60.

The pulse waveform/modulation adjustment part 64 adjusts the pulse waveform of the functional electrical stimulation to the pulse waveform set by the authentication/FES condition setting part 60. The voltage adjustment part 65 adjusts the voltage of the functional electrical stimulation to an arbitrary voltage set by the authentication/FES condition setting part 60.

The functional electrical stimulation signal generation part 66 outputs to the terminal group 41 (42A, 42B), via the communication means 36, electrical signals in which the stimulation frequency, pulse width, pulse waveform/modulation, and voltage have been adjusted by the stimulation frequency adjustment part 62, the pulse width adjustment part 63, the pulse waveform/modulation adjustment part 64, and the voltage adjustment part 65.

Here, as shown in Fig. 7, the biosignal measurement harness 3 is provided with an integrated control part 50 for integrally controlling the biosignal detection part 30 and the stimulation application part 40, and the integrated control part 50 is configured from a voluntary control part 70, an autonomous control part 71 and an optimal stimulation adjustment part 72.

As described above, the integrated control part 50 generates a designation signal for designating which terminal 41 to select from the terminal groups 42A, 42B provided to the harness body 3A, and a signal for the start or end of the acquisition of data, and sends these signals to the measurement module controller 34 via the antenna. The measurement module controller 34 selects and operates the designated terminal 41 according to the received signals.

The voluntary control part 70 within the integrated control part 50 voluntarily controls the stimulation application part 40 so as to generate stimulating power according to the wearer's intention based on the biosignal detected by the biosignal detection part 30. The voluntary control part 70 can also select the optimal combination among a plurality of terminals 41 configuring the terminal groups 42A, 42B in the stimulation application part 40 based on the distribution measurement result of the biosignal of the wearer's body from the biosignal detection part 30 upon voluntarily controlling the stimulation application part 40. The relationship of the distribution measurement result of the biosignal and the terminal 41 selected from the terminal groups 42A, 42B at such point in time may be set in advance according to the wearer's attribute, or may be re-set while making adjustments according to the feedback result of the actual plantar-dorsiflexion motion of the wearer's toes.

Moreover, the autonomous control part 71 autonomously controls the stimulation application part 40 (42A, 42B) so as to cause the ankle to dorsiflex or plantarflex based on the contraction of the wearer's dorsiflexor muscles or plantar flexor muscles based on the wearer's walking cycle obtained from the recognition result of the gait recognition part 22 received from the walking state detection part 2 (Fig. 2) via the communication part 73.

Furthermore, the optimal stimulation adjustment part 72 adjusts the hybrid ratio of the voluntary control by the voluntary control part 70 and the autonomous control by the autonomous control part 71 so that the left/right balance of the wearer's gait recognized by the gait recognition part 22 will fall within a predetermined range.

According to the walking support device 1 of this embodiment, without the wearer having to wear a drive mechanism such as an actuator, it is possible to voluntarily control the stimulation application part 40 so as to generate stimulating power according to the wearer's intention and autonomously control the stimulation application part 40 so as to cause the ankle to dorsiflex or plantarflex based on contraction of the dorsiflexor muscles or the plantar flexor muscles of the wearer based on the wearer's walking cycle.

Furthermore, the wearer can smoothly perform the plantar-dorsiflexion motion of the toes in coordination with the physical stimulating power according to one's own intention while maintaining the left/right balance of one's gait by adjusting the hybrid ratio of voluntary control and autonomous control so that the left-right balance of the wearer's gait falls within a predetermined range.

The stimulation application part 40 described above adjusts at least one among strength level, pattern and time of applying stimulation to the wearer's paralysis site according to control of the voluntary control part 70 and/or the autonomous control part 71. Consequently, with the walking support device 1, adjustment can be made so that the plantar-dorsiflexion motion of the wearer's toes is performed smoothly while maintaining the left/right balance of the wearer's gait at the same time.

### (2) Other embodiments

As explained above, while this embodiment explained a case of configuring the stimulation application part from a device that causes the muscles of the paralysis site to contract based on functional electrical stimulation (FES), the present invention is not limited thereto, and electrical stimulation such as therapeutic electrical stimulation (TES), ultra-high frequency stimulation, ultrasound stimulation and various other types of physical stimulation may be applied so as long as the device is able to apply physical stimulation.

In addition, the present invention may further comprise a sound collecting microphone (not shown) which is provided to the shoe on a side where the wearer's ankle has a paralysis site, and collects sound of footsteps of the shoe, and the optimal stimulation adjustment part 72 (Fig. 7) may correct the hybrid ratio based on the sound of footsteps collected with the sound collecting microphone. Consequently, with the walking support device, by comprehending the status of a floor surface while collecting the sound when the wearer's toes contact the floor surface, adjustment can be made even more accurately so that the plantar-dorsiflexion motion of the wearer's toes is performed smoothly.

In addition, the present invention may further comprise a vibration detection part (not shown) which is provided to the shoe on a side where the wearer's ankle has a paralysis site, and detects vibration from a floor surface while the wearer is walking, and the optimal stimulation adjustment part 72 (Fig. 7) may correct the hybrid ratio based on the vibration detected with the vibration detection part. Consequently, with the walking support device, by comprehending the status of a floor surface while detecting the vibration from the floor surface that reaches the wearer's shoes, adjustment can be made even more accurately so that the plantar-dorsiflexion motion of the wearer's toes is performed smoothly.

### REFERENCE SIGNS LIST

1... walking support device, 2... walking state detection part, 3... biosignal measurement harness, 3A... harness body, 10... load measurement part, 11... foot motion detection part, 12... control unit, 13... data storage part, 14... communication part, 20... center of gravity calculation part, 21... foot movement locus calculation part, 22... gait recognition part, 30... biosignal detection part, 31... electrode, 32A to 32F... electrode group, 33... measurement module, 34... measurement module controller, 40... stimulation application part, 41... terminal, 42A, 42B... terminal group, 50... integrated control part, 70... voluntary control part, 71... autonomous control part, 72... optimal stimulation adjustment part.

## Claims

1. A walking support device, comprising:
a load measurement part which is attached to each inner sole part of a pair of left and right shoes of a wearer, and which measures a load applied to an underside of the wearer's feet;
a center of gravity calculation part which calculates a center of gravity of each of the feet based on a change of each load measured with the load measurement part;
a foot motion detection part which is mounted on a predetermined position of each of the shoes, and detects an acceleration and/or an angular rate during movement of the foot;
a movement locus calculation part which calculates a movement locus of each of the feet based on each acceleration and/or each angular rate detected by the foot motion detection part;
a gait recognition part which recognizes the wearer's gait based on the calculated center of gravity and movement locus of each of the feet;
a biosignal detection part which has an electrode group for detecting the wearer's biosignal each disposed on the wearer's body surface area with reference to a joint associated with the wearer's walking motion;
a stimulation application part which has a terminal group for applying physical stimulation to a body surface of a paralysis site each disposed at the paralysis site centered around dorsiflexor muscles and plantar flexor muscles of the wearer's ankle; a voluntary control part which voluntarily controls the stimulation application part so as to generate stimulating power according to the wearer's intention based on the biosignal detected by the biosignal detection part;
an autonomous control part which autonomously controls the stimulation application part so as to cause the ankle to dorsiflex or plantarflex based on contraction of the dorsiflexor muscles or the plantar flexor muscles of the wearer based on the wearer's walking cycle obtained from a recognition result of the gait recognition part; and
an optimal stimulation adjustment part which adjusts the voluntary control by the voluntary control part and the autonomous control by the autonomous control part so that a left/right balance of the wearer's gait recognized by the gait recognition part will fall within a predetermined range.

2. The walking support device according to claim 1,
wherein the stimulation application part adjusts at least one among strength level, pattern and time of applying stimulation to the wearer's paralysis site according to control of the voluntary control part and/or the autonomous control part.

3. The walking support device according to claim 1 or claim 2, further comprising:
a sound collecting microphone which is provided to the shoe on a side where the wearer's ankle has a paralysis site, and collects sound of footsteps of the shoe,
wherein the optimal stimulation adjustment part corrects the hybrid ratio based on the sound of footsteps collected with the sound collecting microphone.

4. The walking support device according to any one of claims 1 to 3, further comprising:
a vibration detection part which is provided to the shoe on a side where the wearer's ankle has a paralysis site, and detects vibration from a floor surface while the wearer is walking,
wherein the optimal stimulation adjustment part corrects the hybrid ratio based on the vibration detected with the vibration detection part.

5. A walking support method, comprising:
a first process of calculating a center of gravity of each of a wearer's feet based on a change of a load applied to an underside of each of the feet;
a second process of calculating a movement locus of each of the feet based on an acceleration and/or an angular rate during movement of each of the feet;
a third process of recognizing the wearer's gait based on the calculated center of gravity and movement locus of each of the feet;
a fourth process of detecting the wearer's biosignal using an electrode group each disposed on the wearer's body surface area with reference to a joint associated with the wearer's walking motion;
a fifth process of applying physical stimulation to a body surface of a paralysis site each disposed at the paralysis site centered around dorsiflexor muscles and plantar flexor muscles of the wearer's ankle;
a sixth process of voluntarily controlling the application of stimulation in the fifth process so as to generate stimulating power according to the wearer's intention based on the biosignal detected in the fourth process;
a seventh process of autonomously controlling the application of stimulation in the fifth process so as to cause the ankle to dorsiflex or plantarflex based on contraction of the dorsiflexor muscles or the plantar flexor muscles of the wearer based on the wearer's walking cycle obtained from a recognition result of the third process; and
an eighth process of adjusting the voluntary control based on the sixth process and the autonomous control based on the seventh process so that a left/right balance of the wearer's gait recognized in the third process will fall within a predetermined range.

6. The walking support method according to claim 5,
wherein, in the fifth process, at least one among strength level, pattern and time of applying stimulation to the wearer's paralysis site is adjusted according to the voluntary control in the sixth process and/or the autonomous control in the seventh process.

7. The walking support method according to claim 5 or claim 6,
wherein, in the eighth process, the hybrid ratio is corrected based on a sound of footsteps collected from the shoe on a side where the wearer's ankle has a paralysis site.

8. The walking support method according to any one of claims 5 to 7,
wherein, in the eighth process, the hybrid ratio is corrected based on a vibration detected from the shoe on a side where the wearer's ankle has a paralysis site.
